# FASCICULE DE BREVET EUROPEEN

(11) **EP 0 646 003 B1**
(45) Date de publication et mention de la délivrance du brevet: **14.08.1996**
(21) Numéro de dépôt: 93913149.6
(22) Date de dépôt: 16.06.1993
(51) Int. Cl.: A61K 9/51

(54) **PREPARATION ET UTILISATION DE NOUVEAUX SYSTEMES COLLOIDAUX DISPERSIBLES A BASE DE CYCLODEXTRINE, SOUS FORME DE NANOSPHERES**
HERSTELLUNG UND VERWENDUNG VON, AUF BASIS VON CYCLODEXTRIN DISPERGIERBAREN KOLLOIDAL-SYSTEMEN, IN FORM VON NANOKUGELN
PREPARATION AND USE OF NOVEL CYCLODEXTRIN-BASED DISPERSIBLE COLLOIDAL SYSTEMS IN THE FORM OF NANOSPHERES

(30) Priorité: 16.06.1992 FR 9207287
(43) Date de publication de la demande: 05.04.1995
(73) Titulaire: CENTRE NATIONAL DE LA RECHERCHE SCIENTIFIQUE (CNRS), 75007 Paris (FR)
(72) Inventeur: SKIBA, Mohamed, F-91370 Verrières-le-Buisson (FR); WOUESSIDJEWE, Denis, F-92160 Antony (FR); COLEMAN, Antony, F-91640 Briis-sous-Forges (FR); FESSI, Hatem, F-75014 Paris (FR); DEVISSAGUET, Jean-Philippe, F-92200 Neuilly-sur-Seine (FR); DUCHENE, Dominique, F-75116 Paris (FR); PUISIEUX, Francis, F-94700 Maisons-Alfort (FR)
(74) Mandataire: Le Guen, Gérard
(86) Numéro de dépôt international: FR9300594
(87) Numéro de publication internationale: WO9325195

(56) Documents cités:
- EP-A- 0 274 961
- WO-A-84/00294
- FR-A- 2 551 072

## Description

La présente invention a pour objet la préparation et l'application d'un nouveau système colloïdal dispersible à base de cyclodextrine sous forme de particules sphériques de type matriciel et de taille allant de 90 à 900 nm (nanosphères), pouvant contenir une molécule active.

FR 2 551 072 décrit des capsules micrométriques comme forme pharmaceutique à libération prolongée, preparées à partir d'esters de polyol. Ces micro-particules ne sont pas adaptées à une administration intra-vasculaire sans risques d'embolisation.

Des particules submicroniques sont déjà connues notamment d'après BE-A-808 034, BE-A-839 748. BE-A-869 107, FR-A-2 504 408, EP-A-02 75 796 et EP-A-03 49 428.

Ainsi, les brevets belges n°808 034 et 839 748 décrivent des particules submicroniques formées de matières polymérisables comme les dérivés de l'acide acrylique ou méthacrylique par exemple le méthacrylate de méthyle ou de butyle, le méthacrylamide ou un mélange de ces composés. Les particules submicroniques formées par polymérisation micellaire de ces différents monomères ont à la fois la propriété d'enrober totalement ou partiellement la substance biologiquement active et la propriété de former des suspensions aqueuses colloïdales qui permettent une administration parentérale de ces particules ainsi chargées de molécules bioloqiquement actives.

Cependant, les polymères de dérivés de l'acide acrylique ou méthacrylique décrits dans ces brevets pour la préparation de particules submicroniques, contenant une molécule biologiquement active, sont substantiellement stables de sorte qu'ils subsistent longtemps tels quels dans les tissus ou dans la cavité où ils ont été administrés et ceci constitue un inconvénient, plus particulièrement dans le cas d'administration parentérale en médecine humaine.

Le brevet belge n°869 107 remédie à ce désavantage en décrivant des nanoparticules biodégradables contenant une molécule biologiquement active. Le matériau utilisé est constitué par des polymères de cyanoacrylate d'alkyles déjà utilisés en chirurgie comme adhésifs tissulaires et connus pour leur biodégradabilité.

Son inconvénient majeur tient à la structure des particules obtenues, à la toxicité des produits de dégradation et au mode d'incorporation de la substance active. Le mode opératoire décrit permet seulement de préparer des particules formées d'un réseau polymérique très dense. La molécule active ne peut être incoporée que par adsorption et le taux fixé est toujours relativement faible. De plus il est difficile de maîtriser le poids moléculaire du polymère constitutif des nanoparticules et il est nécessaire, notamment en vue d'usages biologiques, d'éliminer des monomères et oligomères résiduels, le cas échéant les réactifs de polymérisation (initiateur et catalyseur) en excès ainsi que les surfactifs s'ils sont utilisés à forte concentration ou s'ils ne sont pas biocompatibles. Or, la purification (ultracentrifugation, dialyse) s'avère souvent lourde car la filtration des nanoparticules, vu leur taille, n'est pas toujours possible.

On a aussi proposé des nanoparticules à base de protéines, notamment par dénaturation à chaud d'une émulsion eau dans l'huile d'une solution protéique telle que l'albumine, (Kramer, P.A : J.Phar.Sci., 63 1646, 1974) ou par désolvatation d'une solution protéique, telle que la gélatine, par un sel minéral ou l'ethanol (Marty et coll., Austr. J.Pharm. Sci., 6, 65, 1978, ou Pharm. Acta Helv. 1, 53, 1978), puis, dans les deux cas, par durcissement au moyen d'un aldéhyde. Les nanoparticules selon Kramer ont pour inconvénient principal de nécessiter une émulsification préalable de la solution aqueuse de la matière première macromoléculaire dans une phase continue huileuse. Cette émulsion devant être très fine, l'utilisation de surfactifs et d'appareillages adhoc (sonicateur, etc) est indispensable pour obtenir des nanoparticules de taille convenable. Quant aux nanoparticules selon Marty, elles font appel à des quantités importantes de sels minéraux qu'il faut éliminer, de même que l'excès d'aldéhyde et de sulfite ou de métabisulfite utilisés pour neutraliser ce dernier.

EP-A-02 75 796 et -03 49 428 décrivent des procédés d'obtention de nanoparticules d'une substance par une méthode de désolvatation consistant à mélanger deux phases de solvants, l'un étant non-solvant de la substance mais soluble dans l'autre solvant.

Les nanoparticules selon EP-A-03 49 428 sont préparées à certaines conditions c'est-à-dire, la température du solvant doit être inférieure à la température de coagulation de la protéine et le pH du non-solvant doit être éloigné du point isoélectrique de la protéine.

Selon la présente invention on propose comme matière de base des nanosphères, des cyclodextrines modifiées préparées par acylation de cyclodextrines naturelles. Elles ont l'avantage d'être biodégradables, leur administration est suivie d'une libération de la molécule active, et il est possible d'obtenir une biodégradabilité, convenablement programmée en faisant appel à des cyclodextrines modifiées différant entre elles par la nature de la chaîne alkyle du groupe acyle utilisé. De telles cyclodextrines modifiées et leur préparation sont notamment décrits par Ping Zhang, Chang-Chung Ling, A.W. Coleman, Parrot-Lopez et H.Galons dans Tetr. Lett. 32, N°24, 2769-70, 1991.

Ainsi l'invention a pour objet un procédé de préparation d'un système colloïdal dispersible à base de cyclodextrine sous forme de nanosphères caractérisée en ce que :
1) on prépare une phase liquide constituée par une solution de cyclodextrine modifiée par des groupes acyle dans un solvant ou mélange de solvants organique (s), choisi(s) parmi le méthanol, l'éthanol, l'isopropanol et l'acétone, contenant ou non un surfactif et pouvant être additionnée d'une molécule active,
2) on prépare une seconde phase liquide constituée par de l'eau ou un mélange aqueux, contenant ou non un surfactif et pouvant être additionnée d'une molécule active, et
3) on ajoute sous agitation modérée, l'une des phases liquides obtenues sous (1) ou (2) à l'autre, de manière à obtenir pratiquement instantanément une suspension colloïdale de nanosphères de cyclodextrine modifiée contenant le cas échéant ladite molécule active.

Si l'on désire, on peut éliminer tout ou une partie du solvant ou du mélange de solvants et de l'eau ou du mélange aqueux, de manière à obtenir une suspension colloïdale de concentration voulue en nanosphères ou une poudre de nanosphères.

La cyclodextrine modifiée utilisée selon l'invention est notamment une cyclodextrine dont les groupes hydroxy, de préférence les hydroxy secondaires de chaque unité glucose la formant ont été estérifiés par un groupe acyle aliphatique ou aromatique pouvant être substitués par un ou plusieurs groupements fonctionnels, telle qu'une béta-cyclodextrine acylée par un groupe alcanoyle de 2 à 20 atomes de carbone, notamment 6 à 14 atomes de carbone. Ces produits sont décrits par Ping Zhang et coll. précité.

La molécule active est ajoutée de préférence à la phase dans laquelle elle est soluble, notamment à la phase (1) si elle est liposoluble ou à la phase (2) si elle est hydrosoluble.

La molécule active peut être un principe médicamenteux, un réactif biologique, un principe cosmétique, ou un produit chimique. L'invention permet d'obtenir des nanosphères de cyclodextrine modifiées seules (utilisables telles quelles) ou comprenant cette molécule active (emprisonnée dans sa structure).

Le solvant organique dans la phase (1) peut être un alcool tel que méthanol, éthanol, isopropanol, etc... ou une cétone telle que l'acétone.

L'eau ou un mélange aqueux (eau salée, acidifiée, alcalinisée, etc...) est le non-solvant dans la phase (2).

Le procédé peut être réalisé à différentes températures (qui influent peu sur son déroulement), notamment entre 0°C et la température d'ébullition des solvants. Le rapport des volumes phase (1)/phase (2) peut varier préférentiellement de 0,1 à 1.

Le(s) surfactif(s) est (sont) présent(s) notamment en une proportion de 0,1 à 10%, de préférence 0,2 à 2% en poids de la suspension colloïdale obtenue dans l'étape 3.

On entend par agitation modérée une agitation suffisante pour homogénéiser le mélange des phases (1) et (2), par exemple au moyen d'un barreau magnétique à 50-500 tr/min, par exemple 100 tr/min. Elle n'est pas indispensable pour de faibles quantités de produit.

Finalement, la suspension colloïdale de nanosphères peut être à volonté concentrée, stérilisée, tamponnée (par exemple au pH physiologique), et lyophilisée. Pour les sphères obtenues selon la présente invention, la préparation a l'avantage d'être réversible: on peut solubiliser les nanosphères et repréparer les nanosphères à partir de cette solution suivant le mode opératoire. De plus les suspensions de nanosphères présentent une grande stabilité avec le temps.

Les nanosphères obtenues suivant l'invention se présentent au microscope électronique à transmission, après coloration négative avec l'acide phosphotungstique, sous la forme de particules non contrastées sensiblement sphériques et, après cryofracture sous la forme de particules sphériques denses de type matriciel.

Selon les conditions opératoires, il est possible d'obtenir des nanosphères dont le diamètre varie d'environ 90 à environ 900 nanomètres, de préférence de 90 à 300 nm, notamment 150 à 300 nm.

Les nanosphères obtenues de l'invention peuvent contenir au sein de leur réseau une molécule active par exemple une molécule médicamenteuse à usage humain ou vétérinaire ou un produit pour le diagnostic. Comme molécule médicamenteuse, on peut citer plus particulièrement les produits chimiques doués de propriétés pharmacologiques et par exemple, les substances antimitotiques ou antinéoplasiques comme la méthotréxate, l'actinomycine D, l'adriamycine, la daunorubicine, la bléomycine, et la vincristine ou les substances antibiotiques comme les pénicillines, les céphalosphorines et l'acide nalidixique, les antibiotiques du type aminoglycoside et ceux de la famille de la virginiamycine et les substances hormonales, notamment les hormones stéroïdiennes. Ces molécules médicamenteuses peuvent être notamment des composés chimiques à haut poids moléculaire comme l'insuline et l'héparine et l'expression "molècule médicamenteuse" comprend également des produits biologiques comme les antigènes, les enzymes, les protéines, les virus ou des constituants de virus, de bactéries ou de cellules. Les nanosphères suivant l'invention peuvent également contenir un produit pour le diagnostic comme par exemple la fluorescéine et la séralbumine humaine radio-active.

En médecine humaine ou vétérinaire, les nanosphères de l'invention peuvent être ultilisés comme vecteurs de médicaments administrés avec ou sans excipient adéquat par voie orale, sous cutanée, intradermique, intramusculaire ou intraveineuse et leur diffusion dans les tissus les rend particulièrement intéressantes pour les traitments par voie générale.

Les nanosphères de la présente invention, contrairement aux nanoparticules formées d'un réseau dense, présentent l'avantage de permettre un taux d'incorporation nettement plus élevé. Ceci est dû à la possibilité de double charge, en premier lieu, une charge dans le réseau et en second lieu dans la cavité de la cyclodextrine à condition que la molécule invitée ait une conformation convenable par rapport à la cavité.

L'invention est illustrée par les exemples suivants :

### Exemple 1

Préparation de nanosphères de cyclodextrine modifiée à 6 carbone.

On utilise une béta-cyclodextrine dont les OH secondaires des unités glucose la formant ont été extérifiés par des groupes hexanoyle, préparée selon Ping Zhang et coll.

| Phase 1 | |
|---|---|
| béta-cyclodextrine modifiée à 6 C | 50 mg |
| acétone | 50 ml |

| Phase 2 | |
|---|---|
| Pluronic (R) F68 | 62,5 mg |
| eau déminéralisée ou distillée | 25 ml |

La phase 1 est ajoutée sous agitation magnétique à la phase 2. Le milieu devient immédiatement opalescent par formation de nanosphères de cyclodextrine modifiée. La taille moyenne des nanosphères mesurée par un diffractomètre à rayon laser (Nanosizer (R) de chez Coultronics) est de 180 nm avec un indice moyen de dispersion de 0,08.

La suspension peut être concentrée sous pression réduite au volume désiré, par exemple 5 ml ou plus ou moins.

Après un repos prolongé (14 mois), l'aspect de la suspension de nanosphères demeure inchangé et on n'observe, en particulier, ni sédimentation irréversible, ni variation de la taille des nanosphères.

### Exemple 2 : (variante de l'exemple 1)

On procède comme dans l'exemple 1, mais en ajoutant la phase aqueuse à la phase acétonique. Les nanosphères obtenus présentent les mêmes caractéristiques que dans l'exemple 1.

### Exemple 3 : (variante de l'exemple 1)

On procède comme dans l'exemple 1, mais en ajoutant la phase acétonique à la phase aqueuse sans agitation du milieu. Les nanosphères obtenues ont une taille de 200 nm avec un indice moyen de dispersion 0,5.

### Exemple 4 : (variante de l'exemple 1)

On procède comme dans l'exemple 1, mais sans ajouter d'agent de surface à la phase aqueuse. Les nanosphères obtenues ont une taille de 200 nm avec un indice moyen de dispersion de 0,6.

### Exemple 5

Préparation stérile de nanosphères de cyclodextrine modifiée à 6 carbone. On procède comme dans l'exemple 1, puis la suspension est stérilisée à l'autoclave à 120°C pendant 15 minutes. La taille moyenne des particules demeure pratiquement inchangée aprés stérilisation.

### Exemple 6

Préparation lyophilisée de nanosphères de cyclodextrine modifiée à 6 carbone.

On procède comme dans l'exemple 1, puis la suspension est lyophilisée. L'addition d'un cryoprotecteur (maltose, tréhalose...etc) n'est pas indispensable. La taille moyenne des particules mesurée juste après la lyophilisation demeure inchangée.

### Exemple 7

Préparation de nanosphères de cyclodextrine modifiée à 12 carbone.

On procède comme dans l'exemple 1, en remplaçant la cyclodextrine modifiée à 6 carbone par la cyclodextrine modifiée à 12 carbone c'est-à-dire une beta-cyclodextrine acylée par des groupes dodecanoyle. La taille moyenne des nanosphères est de 172 nm avec un indice moyen de dispersion de 0,1.
Ces nanosphères peuvent être stérilisées à l'autoclave et lyophilisées comme celles avec 6 carbone.

### Exemple 8

Préparation de nanosphères de cyclodextrine modifiée à 14 carbone.

On procède comme dans l'exemple 1, en remplaçant la cyclodextrine modifiée à 6 carbone par la cyclodextrine modifiée à 14 carbone, c'est-à-dire acylée par des groupes tétradécanoyle. La taille moyenne des nanosphères est de 110 nm avec un indice moyen de dispersion de 0,1.
Les nanosphères de cyclodextrine modifiée à 14 carbone peuvent être stérilisées à l'autoclave et lyophilisés comme celles avec 6 carbone.

### Exemple 9

Stabilité des nanosphères de cyclodextrine en présence de forces ioniques variables.
On procède comme indiqué dans l'exemple 1.
Après concentration de la suspension de nanosphères de cyclodextrine modifiée jusqu'à un volume de 10 ml, on ajoute progressivement à celle-ci des quantités croissantes de chlorure de sodium. La suspension de nanosphères est parfaitment stable lorsque la concentration en chlorure de sodium correspond à l'isotonie avec le sang et le demeure jusqu'à une concentration supérieure à 3 fois la concentration isotonique.

### Exemple 10

Stabilité des nanosphères de cyclodextrine en présence d'un milieu acide ou basique.
On procède comme indiqué dans l'exemple 1.
Après concentration de la suspension de nanosphères de cyclodextrine jusqu'à un volume de 10 ml, on ajoute progressivement à celle-ci des quantités croissantes d'acide chlorydrique (1N) ou de la soude (1N). La suspension de nanosphères est parfaitement stable.

### Exemple 11

Stabilité des nanosphères de cycodextrine à la température.
On procède comme indiqué dans l'exemple 1.
Après concentration de la suspension de nanosphères d e cyclodextrine jusqu'à un volume de 10 ml, on place chaque lot à 4°C, 25°C et 40°C.
Les suspensions demeurent stables dans le temps et ne présentent, après 14 mois de conservation, ni sédimentation irréversible, ni variation de la taille des nanosphères.

### Exemple 12

Préparation de nanosphères en présence d'un sel.
On procède comme indiqué dans l'exemple 1, mais la phase aqueuse est aditionnée de 90 mg de chlorure de sodium. Après concentration de la suspension de nanoparticules jusqu'à un volume de 10 ml, correspondant compte tenu du chlorure de sodium à l'isotonie avec le sang, les nanosphères ont une taille moyenne de 200 nm avec un indice moyen de dispersion de 1.
La suspension demeure stable dans le temps et ne présente, après 14 mois de conservation, ni sédimentation irréversible, ni variation de la taille des nanoparticules.

### Exemple 13

Addition de non-solvant dans la phase du solvant.
On procède comme dans l'exemple 1, mais la cyclodextrine est dissoute dans un mélange acétone/eau (90/10, v/v), au lieu d'acétone pure. La présence d'une faible proportion de non-solvant de la cyclodextrine dans un solvant, conduit à des nanosphères dont la taille moyenne est de 180 nm avec un indice moyen de dispersion de 0,5.

### Exemple 14

Stabilité des nanosphères de cyclodextrine aux ultrasons.
On procède comme dans l'exemple 1. Après concentration de la suspension de nanosphères de cyclodextrine jusqu'à un volume de 10 ml, on place la suspension de nanosphères de cyclodextrine dans un bain à ultrasons pendant trois heures.
La suspension demeure stable dans le temps et ne présente, après 14 mois de conservation, ni sédimentation irréversible, ni variation de la taille des nanosphères.

### Exemple 15

Préparation de nanosphères en présence d'un principe actif lipophile.
On procède comme dans l'exemple 1, mais on ajoute 20 mg d'indométacine dans la phase acétonique. Les nanosphères obtenues ont une taille moyenne de 200 nm avec un indice de dispersion de 0,5. Après ultracentrifugation et dosage de l'indomécatine dans la phase dispersante, la quantité de principe actif incorporé dans les nanosphères représente 70% de la quantité initiale.

### Exemple 16

Préparation de nanosphères contenant de la doxorubicine.
On procède comme dans l'exemple 1, mais on ajoute 5 mg de doxorubicine dans la phase aqueuse. Les nanosphères obtenues ont une taille moyenne de 200 nm et un indice moyen de dispersion de 1. Après ultracentrifugation et dosage de la doxorubicine dans la phase dispersante, la quantité de principe actif incorporé dans les nanosphères représente 60% de la quantité initiale.

### Exemple 17

Préparation de nanosphères contenant de la progestérone.
On procède corne dans l'exemple 1, mais on ajoute 150 mg de progestérone dans la phase 1. Les nanosphères obtenues ont une taille moyenne de 120 nm et un indice de dispersion de 0,2. Après ultracentrifugation et dosage de la progestérone dans la phase dispersante, la quantité de principe actif incorporé dans les nanosphères représente 60% de la quantité initiale.

### Exemple 18

Préparation de nanosphères contenant de l'amphotéricine B.
On procède comme dans l'exemple 1, mais on ajoute 6 mg d'amphotéricine B dans la phase 1. Les nanosphères obtenues ont une taille moyenne de 180 nm et un indice de dispersion de 0,2. Après ultracentrifugation et dosage de l'amphotéricine dans la phase dispersante, la quantité de principe actif incorporé dans les nanosphères représente 90% de la quantité initiale.

### Exemple 19

Préparation de nanosphères contenant un colorant lipophile, le soudan III.
On procède comme dans l'exemple 1, mais on ajoute 5 mg de soudan III dans la phase 1. Une faible quantité est précipitée et reste sur le filtre. Les nanosphères obtenues ont une taille moyenne de 130 nm et un indice de dispersion de 0,2.

Les nanosphères obtenues selon l'invention peuvent trouver des applications dans de nombreux secteurs techniques.

En tant que "**vecteurs**" de principe actif, en thérapeutique humaine et animale les nanosphères permettent d'envisager :
d'atteindre de nouveau sites d'action, en particulier intracellulaires, voire intralysosomiaux ;
d'utiliser de nouvelles voies d'administration pour les principes actifs connus, en augmentant la stabilité et/ou l'absorption des principes actifs, ou en permettant la réalisation de formes injectables par voie intravasculaire, de principes actifs insolubles ;
de modifier la distribution tissulaire des principes actifs, par un meilleur ciblage vers des sites d'actions favorables et/ou un détournement des sites d'effets indésirables voire toxiques (amélioration de l'index thérapeutique).

En pharmacie, ces dispersions colloïdales de cyclodextrine peuvent permettre notamment :
de réaliser des formes injectables de médicaments insolubles,
de stabiliser un principe actif médicamenteux.

Dans le domaine de la phytopharmacie les nanosphères peuvent véhiculer des insecticides, des pesticides, etc. Leur taille peut permettre d'envisager une action plus puissante par une meilleure pénétration à travers la cuticule. La faible viscosité de la dispersion autorise une pulvérisation très facile sous forme de gouttelettes de très petite taille plus efficaces car plus couvrantes.

En cosmétologie, les nanosphères de cyclodextrine peuvent transporter des produits anti-radicalaires ou autres au niveau du derme.

Dans le domaine des peintures, vernis et traitements des surfaces d'une manière générale, les nanosphères permettent de véhiculer des pigments, des réactifs, des décapants sous forme de dispersion aqueuse de très faible viscosité, aisée à pulvériser ou à appliquer, et qui peut, si nécessaire, être rendue visqueuse, voire adhésive (remise en suspension des nanosphères dans un véhicule approprié). La taille réduite des nanosphères conduit à une très grande finesse du dépôt et à une très grande homogénéité, par exemple de pigmentation.

Les nanosphères obtenues selon l'invention peuvent être utilisées dans les domaines de l'imprimerie et de la reprographie, du traitement de surface des textiles et des fibres, ou autre, de la photographie, de la lubrification, de l'agriculture.

## Revendications

1. Procédé de préparation d'un système colloïdal dispersible à base de cyclodextrine sous forme de nanosphères, caractérisé en ce que
1) on prépare une phase liquide constituée par une solution de cyclodextrine modifiée par des groupes acyle dans un solvant ou mélange de solvants organique(s), choisi(s) parmi le méthanol, l'éthanol, l'isopropanol et l'acétone, contenant ou non un surfactif et pouvant être additionnée d'une molécule active,
2) on prépare une seconde phase liquide constituée par de l'eau ou un mélange aqueux, contenant ou non un surfactif et pouvant être additionnée d'une molécule active, et
3) on ajoute l'une des phases liquides obtenues sous (1) ou (2) à l'autre en maintenant une agitation suffisante pour homogénéiser le mélange des phases (1) et (2), de manière à obtenir immédiatement une suspension colloïdale de nanosphères de cyclodextrine modifiée contenant le cas échéant ladite molécule active.

2. Procédé selon la revendication 1, caractérisé en ce que l'on élimine tout ou partie du solvant ou du mélange de solvants et de l'eau ou du mélange aqueux de manière à obtenir une suspension colloïdale de concentration voulue en nanosphères ou à obtenir une poudre de nanosphères.

3. Procédé selon la revendication 1 ou 2, caractérisé en ce que ledit solvant de la phase (1) est choisi parmi les alcools et les cétones ou leurs mélanges.

4. Procédé selon la revendication 1, caractérisé en ce que la β-cyclodextrine modifier est une β-cyclodextrine acylée par un groupe acyle aliphatique ou aromatique.

5. Procédé selon la revendication 4, caractérisé en ce que la cyclodextrine modifiée est une béta-cyclodextrine estérifiée par des groupes alcanoyle de 2 à 20 atomes de carbone.

6. Procédé selon la revendication 5, caractérisé en ce que la cyclodextrine modifiée est une béta-cyclodextrine estérifiée par des groupes alcanoyle de 6 à 14 atomes de carbone.

7. Procédé selon l'une des revendications 1 à 6, caractérisé en ce que que la molécule active est un principe actif médicamenteux ou un précurseur médicamenteux à usage humain ou vétérinaire, un réactif biologique ou un principe cosmétique, un virus, un constituant de virus, de bactérie ou de cellule, un antigène, un allergène ou une enzyme.

8. Procédé selon l'une quelconque des revendications 1 à 7, caractérisé en ce que le rapport des volumes phase (1)/phase (2) est de 0,1 à 1.

9. Procédé selon la revendication 2, caractérisé en ce que la totalité de l'eau est éliminée par lyophilisation.

10. Procédé selon l'une quelconque des revendications 1 à 9, caractérisé en ce que le(s) surfactif(s) est (sont) présent(s) en une proportion de 0,1 à 10%, de préférence 0,2 à 2% en poids de la suspension colloïdale obtenue dans l'étape 3.

11. Utilisation des nanosphères de cyclodextrine modifiée présentant un diamètre compris entre 90 et 900 nm obtenues suivant l'une quelconque des revendications 1 à 10 comme vecteurs de principes actifs médicamenteux ou cosmétiques.

12. Utilisation des nanosphères de cyclodextrine modifiée présentant un diamètre compris entre 90 et 900 nm obtenues suivant l'une quelconque des revendications 1 à 10 comme vecteurs de produits chimiques.

## Claims

1. Process for the preparation of a dispersible colloidal system based on cyclodextrin in the form of nanospheres, characterised in that
1) a liquid phase is prepared which comprises a solution of cyclodextrin modified by acyl groups in an organic solvent or mixture of organic solvents which is (are) selected from among methanol, ethanol, isopropanol and acetone, which may or may not contain a surfactant and which is capable of accepting an active molecule,
2) a second liquid phase is prepared which comprises water or an aqueous mixture, which may or may not contain a surfactant and which is capable of accepting an active molecule, and
3) one of the liquid phases obtained under (1) or (2) is added to the other, with sufficient stirring maintained to homogenise the mixture of the phases (1) and (2), such as to obtain immediately a colloidal suspension of modified cyclodextrin nanospheres where appropriate containing the said active molecule.

2. Process according to Claim 1, characterised in that all or part of the solvent or mixture of solvents and of the water or aqueous mixture is removed such as to obtain a colloidal suspension of the desired nanosphere concentration or to obtain a nanosphere powder.

3. Process according to Claim 1 or 2, characterised in that the said solvent of the phase (1) is selected from among alcohols and ketones or mixtures thereof.

4. Process according to Claim 1, characterised in that the modified β-cyclodextrin is a β-cyclodextrin which is acylated by an aliphatic or aromatic acyl group.

5. Process according to Claim 4, characterised in that the modified cyclodextrin is a beta-cyclodextrin which is esterified by alkanoyl groups having 2 to 20 carbon atoms.

6. Process according to Claim 5, characterised in that the modified cyclodextrin is a beta-cyclodextrin esterified by alkanoyl groups having 6 to 14 carbon atoms.

7. Process according to one of Claims 1 to 6, characterised in that the active molecule is a medicinal active principle or a medicinal precursor for human or veterinary use, a biological reagent or a cosmetic principle, a virus, a constituent of a virus, bacterium or cell, an antigen, an allergen or an enzyme.

8. Process according to one of Claims 1 to 7, characterised in that the volumetric ratio of phase (1)/phase (2) is from 0.1 to 1.

9. Process according to Claim 2, characterised in that the water is removed in its entirety by lyophilisation.

10. Process according to one of Claims 1 to 9, characterised in that the surfactant(s) is (are) present in a proportion of from 0.1 to 10 wt-%, preferably from 0.2 to 2 wt-%, of the colloidal suspension obtained in stage 3.

11. Use of the nanospheres of modified cyclodextrin exhibiting a diameter within the range 90 to 900 nm obtained in accordance with one of Claims 1 to 10 as vectors of medicinal or cosmetic active principles.

12. Use of the nanospheres of modified cyclodextrin exhibiting a diameter within the range 90 to 900 nm obtained in accordance with one of Claims 1 to 10 as vectors of chemicals.

## Patentansprüche

1. Verfahren zur Herstellung eines dispergierbaren kolloidalen Systems auf der Grundlage von Cyclodextrin in Form von Nanokugeln, **dadurch gekennzeichnet,** daß man
1) eine flüssige Phase bereitet, die aus einer Lösung von mit Acylgruppen modifiziertem Cyclodextrin in einem organischen Lösungsmittel oder einer Mischung von organischen Lösungsmitteln, ausgewählt aus Methanol. Ethanol, Isopropanol und Aceton gebildet ist, bereitet, welche gegebenenfalls ein oberflächenaktives Mittel enthält und mit einem aktiven Molekül versetzt werden kann.
2) eine zweite flüssige Phase, die aus Wasser oder einer wäßrigen Mischung gebildet ist, bereitet, die gegebenenfalls ein oberflächenaktives Mittel enthält und mit einem aktiven Molekül versetzt werden kann, und
3) eine der gemäß (1) oder (2) erhaltenen flüssigen Phasen zu der anderen gibt unter ausreichender Bewegung zur Homogenisierung der Mischung der Phasen (1) und (2), so daß man sofort eine kolloidale Suspension von Nanokugeln von modifiziertem Cyclodextrin erhält, die gegebenenfalls das aktive Molekül enthalten.

2. Verfahren nach Anspruch 1, **dadurch gekennzeichnet,** daß man die Gesamtmenge oder einen Teil des Lösungsmittels oder der Lösungsmittelmischung und des Wassers oder der wäßrigen Mischung entfernt, so daß man eine kolloidale Suspension mit der angestrebten Konzentration von Nanokugeln oder ein Nanokugelpulver erhält.

3. Verfahren nach Anspruch 1 oder 2, **dadurch gekennzeichnet,** daß das Lösungsmittel der Phase (1) aus Alkoholen und Ketonen oder Mischungen davon ausgewählt wird.

4. Verfahren nach Anspruch 1, **dadurch gekennzeichnet,** daß das modifizierte β-Cyclodextrin ein mit einer aliphatischen oder aromatischen Acylgruppe acyliertes β-Cyclodextrin ist.

5. Verfahren nach Anspruch 4, **dadurch gekennzeichnet,** daß das modifizierte Cyclodextrin ein mit Alkanoylgruppen mit 2 bis 20 Kohlenstoffatomen verestertes β-Cyclodextrin ist.

6. Verfahren nach Anspruch 5, **dadurch gekennzeichnet,** daß das modifizierte Cyclodextrin ein mit Alkanoylgruppen mit 6 bis 14 Kohlenstoffatomen verestertes β-Cyclodextrin ist.

7. Verfahren nach einem der Ansprüche 1 bis 6, **dadurch gekennzeichnet,** daß das aktive Molekül ein Arzneimittelwirkstoff oder ein Arzneimittelvorläufer für den menschlichen oder veterinärmedizinischen Gebrauch, ein biologisches Reagens oder ein kosmetischer Wirkstoff, ein Virus, ein Virusbestandteil, ein Bakterium oder eine Zelle, ein Antigen, ein Allergen oder ein Enzym ist.

8. Verfahren nach einem der Ansprüche 1 bis 7, **dadurch gekennzeichnet,** daß das Volumenverhältnis von Phase (1) zu Phase (2)0,1 bis 1 beträgt.

9. Verfahren nach Anspruch 2, **dadurch gekennzeichnet,** daß die Gesamtmenge des Wassers durch Gefriertrocknen entfernt wird.

10. Verfahren nach einem der Ansprüche 1 bis 9, **dadurch gekennzeichnet,** daß das (die) oberflächenaktive(n) Mittel in einer Menge von 0,1 bis 10 Gew.-%, vorzugsweise 0,2 bis 2 Gew.-%, bezogen auf die in der Stufe 3 erhaltene kolloidale Suspension, vorhanden ist (sind).

11. Verwendung der nach einem der Ansprüche 1 bis 10 erhaltenen Nanokugeln aus modifiziertem Cyclodextrin mit einem Durchmesser zwischen 90 und 900 nm als Vektoren für Arzneimittel- oder Kosmetik-Wirkstoffe.

12. Verwendung der nach einem der Ansprüche 1 bis 10 erhaltenen Nanokugeln aus modifiziertem Cyclodextrin mit einem Durchmesser zwischen 90 und 900 nm als Vektoren für chemische Produkte.
